# EUROPEAN PATENT APPLICATION

(11) **EP 4 160 615 A1**
(43) Date of publication of application: **05.04.2023**
(21) Application number: 21306376.1
(22) Date of filing: 01.10.2021
(51) Int. Cl.: G16H 50/20

(54) **METHODS AND SYSTEMS FOR ADMINISTERING COGNITIVE STATE CUES**

(71) Applicant: Société BIC, 92110 Clichy (FR)
(72) Inventor: Duffy, David, 8003 Zurich (CH); Cronin, Harry Michael, Cambridge, CB1 3DE (GB); Wright, Christopher - John, London, E15 4AD (GB); Schnabel, William Andrew, Farnham, Surrey, GU9 8DH (GB)
(74) Representative: Peterreins Schley

(57) **Abstract**

The present invention relates to a computer-implemented method for administering a cognitive state cue to a user of a cognitive task system, comprising obtaining cognitive task data while the user performs a cognitive task; determining the cognitive state cue based on the cognitive task data, thereby generating a cognitive state cue instruction; executing the cognitive state cue instruction, thereby administering the cognitive state cue to the user.

The present invention further relates to a cognitive task system for administering a cognitive state cue to a user of the cognitive task system, comprising: a cognitive task data system configured to obtain cognitive task data while the user performs a cognitive task; a cognitive cue delivery system configured to execute a cognitive state cue instruction; wherein the cognitive state cue instruction is generated by determining the cognitive state cue to be administered to the user based on the cognitive task data.

## Description

### TECHNICAL FIELD

This specification relates to a computer-implemented method and/or a cognitive task system for administering a cognitive state cue to a user of the cognitive task system.

### BACKGROUND

Application of stimuli with different tempo to a person may increase or decrease her/his state of physiological arousal depending on the tempo. Furthermore, stimuli with different tempo may affect the emotional state of the person. In particular, recent studies have shown that slower haptic stimuli (e.g. at a frequency of around 1 Hz) may cause calming effects, i.e. may reduce physiological arousal. Haptics have also been investigated for sustained attention and focus. As an example, sustained attention and focus may be improved by subtle regular haptic cues at 15 Hz and/or active fidgeting activities/behaviors. Fidgets/fidgeting may also be referred to as sensory-motor activities.

Recent fidgeting research has defined four different types of fidgeting behaviors with well-defined physical movement characteristics: relaxing, exploring, active and focus. They have found to be associated with different cognitive benefits.

In the domain of haptics, research has shown that different texture sensations may elicit different emotional sensations. The same is known to be true for music.

Artificial intelligence (AI)/machine learning algorithms assessing the sentiment of small sections of text are known in the art. As a basic example, this may consist of determining positive or negative emotional valence.

AI models and/or autoregressive language models such as GPT-3 are able to generate text based on a small section of text input as a 'seed'. Such algorithms are typically trained on a very large training dataset. Similar AI algorithms may be trained using the output of a specific user to reproduce her/his style.

### SUMMARY

According to a first aspect, there is provided a computer-implemented method for administering a cognitive state cue to a user of a cognitive task system. The method comprises obtaining cognitive task data while the user performs a cognitive task. The method further comprises determining the cognitive state cue to be administered to the user based on the cognitive task data, thereby generating a cognitive state cue instruction. The method further comprises executing the cognitive state cue instruction, thereby administering, via the cognitive task system, the cognitive state cue to the user.

According to a second aspect, there is provided a cognitive task system for administering a cognitive state cue to a user of the cognitive task system. The cognitive task system comprises a cognitive task data system configured to obtain cognitive task data while the user performs a cognitive task. The cognitive task system further comprises a cognitive cue delivery system configured to execute a cognitive state cue instruction, thereby administering the cognitive state cue to the user. The cognitive state cue instruction may be generated by determining the cognitive state cue to be administered to the user based on the cognitive task data. The cognitive task system may be configured to run the computer-implemented method of the first aspect (or an embodiment thereof) for administering a cognitive state cue to the user of the cognitive task system.

According to a third aspect, there is provided a computer system configured to execute the computer-implemented method of the first aspect (or an embodiment thereof) for administering a cognitive state cue to the user of the cognitive task system of the second aspect (or an embodiment thereof). The cognitive task system of the second aspect (or an embodiment thereof) may comprise the computer system of the third aspect (or an embodiment thereof). On the other hand, the computer system of the third aspect (or an embodiment thereof) may comprise the cognitive task system of the second aspect (or an embodiment thereof). For instance, the computer system may comprise a cloud server.

According to a fourth aspect, there is provided a computer program configured to execute the computer-implemented method of the first aspect (or an embodiment thereof) for administering a cognitive state cue to the user of the cognitive task system of the second aspect (or an embodiment thereof).

According to a fifth aspect, there is provided a computer-readable medium or signal storing the computer program of the fourth aspect (or an embodiment thereof).

Dependent embodiments of the aforementioned aspects are given in the dependent claims and explained in the following description, to which the reader should now refer.

Methods and/or systems of the aforementioned aspects of this specification are directed to administering cognitive state cues to a user of the cognitive task system.

A common problem is that (some) people are easily distracted while performing a cognitive task such as e.g. a writing task. Distractions may result from and/or lead to various kinds of micro-breaks. As a result, the quality of the cognitive task output may be negatively affected and/or reduced. On the other hand, (some) people can also become over-focused, which, however, may again reduce the quality of the cognitive task output. Different cognitive tasks and, in particular, different writing tasks may involve very different cognitive processes and therefore benefit from different user cognitive states. In fact, some cognitive tasks rely more on focused attention and others more on diffused and/or broad attention. The problem that is solved by this specification may be phrased as follows: How can we help the user achieve the cognitive state which is beneficial for her/his cognitive task (such as e.g. a writing task)?

As an example, the cognitive task system may comprise a smart pen configured to administer/deliver haptic and/or acoustic stimulus (or other stimulus) to the user which promotes the cognitive state of the user that is required by the cognitive task the user is currently considering and/or performing. The stimulus may be passive such as a haptic rhythm which promotes focus and/or disruption. In this case, the stimulus may merely depend on the cognitive task data (and not e.g. on the user interaction data). On the other hand, the stimulus may be an integrated stimulus with required active and physical user interaction such as e.g. a fidget. The type of fidgeting promotes the required mental and/or emotional state for the cognitive task.

The system may determine an appropriate cognitive state to promote by analyzing the user's cognitive task and compare this to known good states which are associated with different detected cognitive tasks. The cognitive state may be determined continuously and/or multiple times over a period of time. In so doing, a change in the cognitive task may be detected and the stimulus to the user may be adjusted accordingly.

The main steps of the method of the first aspect (or an embodiment thereof) comprise analyzing (the content of) the user's cognitive task to detect the type of cognitive task currently being performed and using the known ideal mental state for the task to administer/deliver appropriate stimulus that maintains and/or promotes a cognitive state beneficial for performing the (current) cognitive task.

The cognitive task system may administer/deliver e.g. rhythmic haptic stimuli that promotes a chosen cognitive state of the user. Where the cognitive task system is to administer/deliver passive rhythmic stimulus to the user, such stimulus may be consciously attended to or unconsciously perceived to affect the cognitive state. Alternatively, or in addition, the cognitive task system may encourage active user interaction which may be performed with or without conscious attention (fidgeting behavior) by delivering patterns of the stimulus that leave e.g. an 'open space' for the user to naturally complete a rhythm. The cognitive task system may be configured to analyze and understand the data generated by a cognitive task system to determine an (currently) ideal cognitive state requirement.

Thanks to the method of the first aspect (or an embodiment thereof) a user's cognitive state is improved in a way which is beneficial to the requirements of a current cognitive task, thereby enabling the user to improve the quality and/or efficiency of her/his output for a range of cognitive tasks. In fact, the user may be distracted from her/his cognitive task less easily by the conscious or subconscious attendance to e.g. rhythmic stimuli. This may be particularly useful for users with attention deficit hyperactivity disorder (ADHD) traits. As a result, the user's focus and/or engagement with the cognitive task may be increased. Furthermore, the user's creativity of her/his cognitive task output may be increased. In fact, the user may be able to 'get into a frame of mind' and/or emotional state more easily using the cognitive task system, for example, enabling her/his to e.g. switch between writing different character's roles more quickly.

The cognitive task system may be enabled to communicate suggested properties of e.g. writing and/or drawing output without communicating specific text or suggestions. This is likely to be seen as less invasive and less likely to cause negative reactions from users that are concerned about their privacy. In other words, it enables the user to be digitally augmented while still retaining creative control.

The cognitive task may be a task which requires mental capabilities to perform. Several types of cognitive tasks may be undertaken by the user of the cognitive task system. For example, the cognitive task may comprise sketching/drawing. Alternatively, or in addition, the cognitive task may comprise note taking. Alternatively, or in addition, the cognitive task may comprise writing. In fact, the cognitive task may comprise creative writing. Alternatively, or in addition the cognitive task may comprise technical subject writing.

The cognitive state cue may be a stimulus which can affect the user's cognitive state in a deterministic and/or probabilistic manner. Such a cognitive state cue may be restricted to a rhythmic cue. The cognitive state cue affects the user's state related to the performance of a cognitive task, in particular physiological arousal (calmness/excitement) and emotional valence (happy/sad).

### FIGURE DESCRIPTION

**Fig. 1a** schematically illustrates a computer-implemented method for administering a cognitive state cue to a user of a cognitive task system.
**Fig. 1b** is a continuation of the schematic illustration of the computer-implemented method for administering a cognitive state cue to a user of a cognitive task system.
**Fig. 1c** schematic illustrates determining the cognitive state cue to be administered to the user based on the cognitive task data.
**Fig. 2** schematically illustrates an example embodiment of the computer-implemented method for administering a cognitive state cue to a user of a cognitive task system.
**Fig. 3** schematically illustrates a cognitive task system for administering a cognitive state cue to a user of the cognitive task system.
**Fig. 4** shows a cognitive task system comprising a digital hand utensil and a user input sensor system.
**Fig. 5** shows an example writing task with two different states required for the cognitive task.
**Fig. 6** shows an example haptic feedback to the user and an example user interaction event.
**Fig. 7a** illustrates an example flow chart for administering a cognitive state cue to a user of a cognitive task system.
**Fig. 7b** illustrates an example flow chart for administering a cognitive state cue to a user of a cognitive task system with user interaction.

### DETAILED DESCRIPTION

There is disclosed a computer-implemented method 100 for administering a cognitive state cue to a user of a cognitive task system 200. The method 100 comprises obtaining 110 cognitive task data while the user performs a cognitive task. The method 100 further comprises determining 130 the cognitive state cue to be administered to the user based on the cognitive task data, thereby generating a cognitive state cue instruction. The method 100 further comprises executing 140 the cognitive state cue instruction, thereby administering, via the cognitive task system 200 or the cognitive cue delivery system 220 thereof, the cognitive state cue to the user.

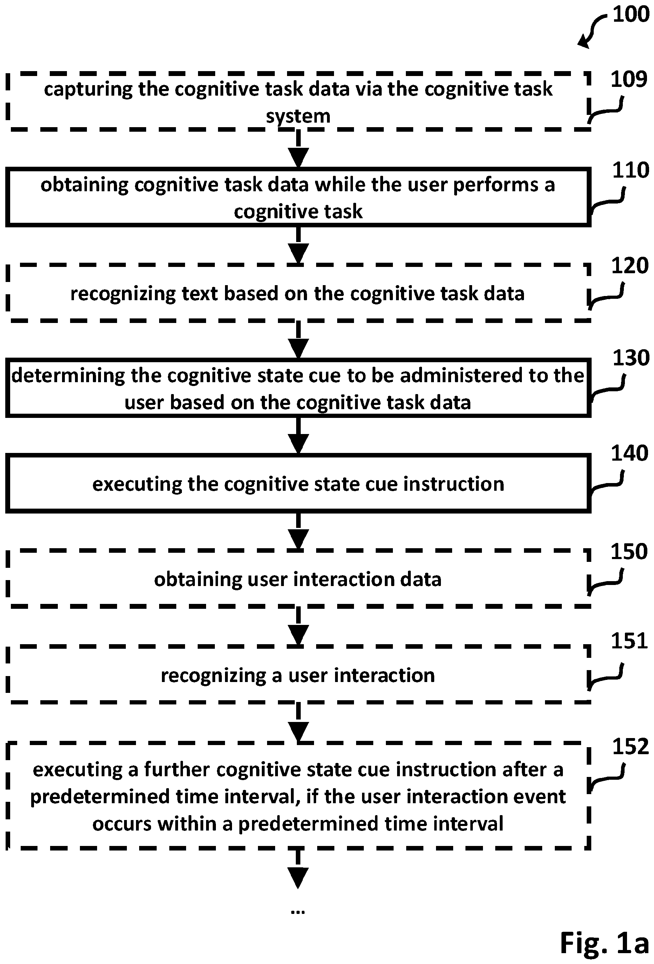

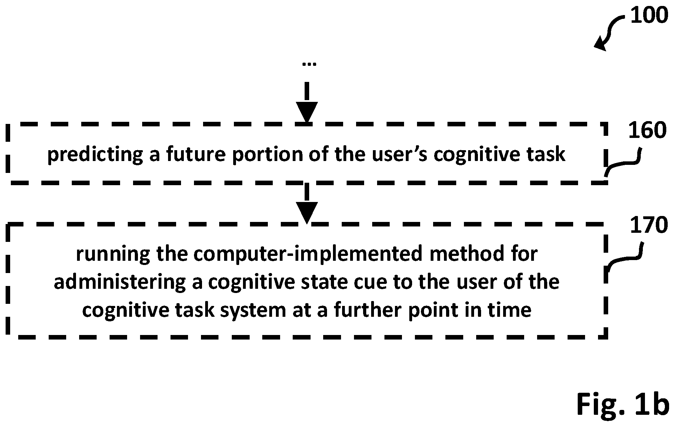

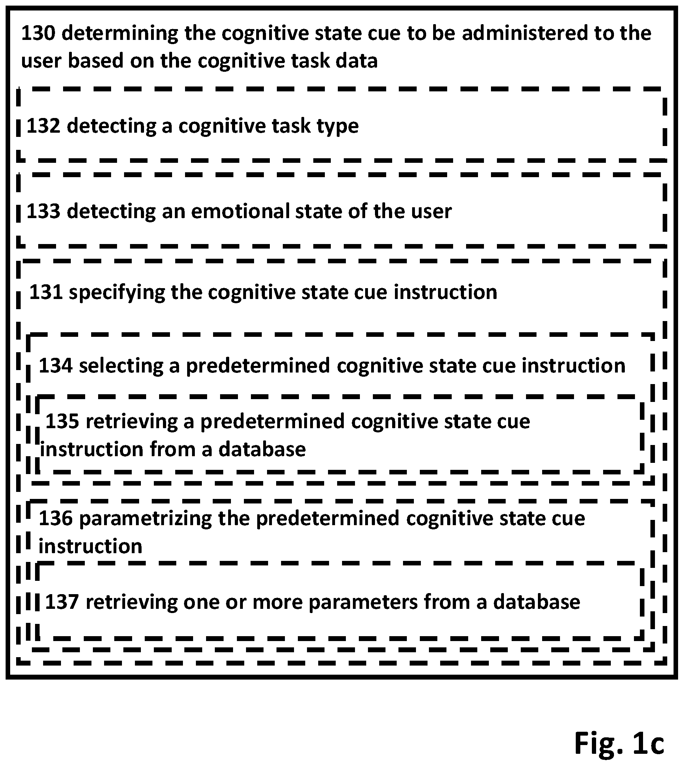

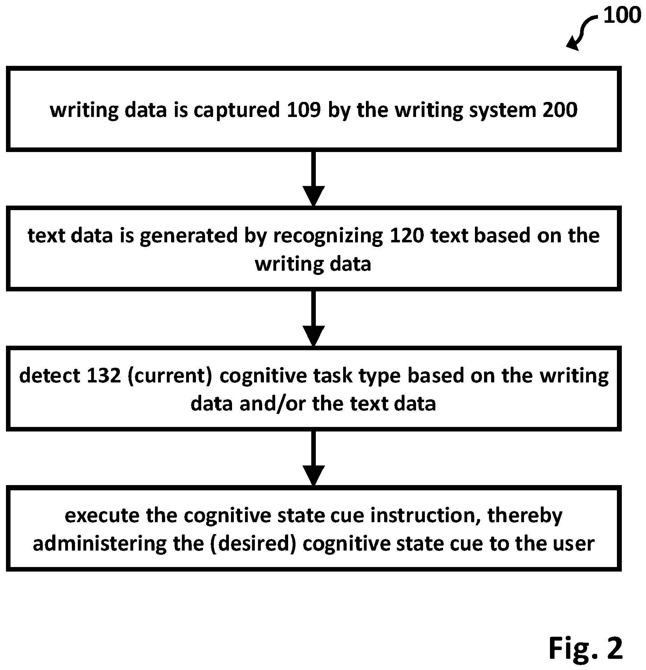

The computer-implemented method 100 is schematically illustrated in **Fig. 1a****-b** (with **Fig. 1b** being a continuation of **Fig. 1a**) and **Fig. 1c**. For example, the method 100 may comprise steps 110, 130, and 140, see e.g. **Fig. 1a****-c.** The method 100 may or may not comprise step 109. The method 100 may or may not comprise step 120. The method 100 may or may not comprise step 150. The method 100 may or may not comprise step 151. The method 100 may or may not comprise step 152. The method 100 may or may not comprise step 160. The method 100 may or may not comprise step 170. In general, the order of steps in **Fig. la-c** shall not be construed as limiting. For instance, step 150 and/or step 160 may be carried out before step 130. An example scenario/embodiment of the method 100 is schematically illustrated in **Fig. 2****.**

The cognitive state cue may comprise a stimulus promoting a cognitive state of the user of the cognitive task system. Administering the cognitive state cue to the user may comprise providing haptic feedback to the user. Alternatively, or in addition, administering the cognitive state cue to the user may comprise providing acoustic feedback to the user. Other feedback to the user may be provided too.

The haptic feedback may comprise a predetermined pattern. For example, the predetermined pattern may comprise a rhythm. Alternatively, or in addition, the predetermined pattern may comprise varying intensities.

The acoustic feedback may comprise a (further) predetermined pattern. For example, the predetermined pattern may comprise a rhythm. Alternatively, or in addition, the predetermined pattern may comprise varying intensities. Alternatively, or in addition, the predetermined pattern may comprise chords. Alternatively, or in addition, the predetermined pattern may comprise a melody, i.e. varying notes and/or chords.

For example, a state required for the cognitive task may be differentiated into two states of 'focused', corresponding to higher physiological arousal, or 'relaxed', corresponding to lower physiological arousal. In general, a plurality of states required for the cognitive task may be differentiated. Each state may correspond to defined stimulus characteristics that may be defined in one or more desired stimulus parameters. For example, the one or more desired stimulus parameters may comprise a pulsed event with the following properties:
i. Stimulus tempo (pulse repetition frequency), with e.g. a relaxed state requiring a lower tempo than a focused stimulus. For example, a tempo of 0.1 Hz to 2 Hz is expected to reduce physiological arousal, whereas a faster tempo of 2-30 Hz may be more suited e.g. for arousing/encouraging the user. These values are based on previous research.
ii. Pulse frequency spectrum. The pulse may e.g. consist of a single or a superposition of sine waves. Properties such as kurtosis may be considered to define specific acoustic properties which have different effects on the noticeability of the stimulus or on the user's cognitive state. Frequency spectrum may influence emotional valence of the stimulus. For haptics, high kurtosis stimuli (which is perceived as rough) may be perceived as negative valence, and pure tones (which may produce a smoother sensation) are perceived as positive emotional valence. Furthermore, chords may be used in acoustic/auditory stimuli which are widely associated with having different emotional valence. It is expected e.g. to use minor chords for negative valence and major chords for positive valence.
iii. Amplitude.
iv. Duration. The duration is expected may e.g. be continuous such that the currently delivered cognitive state cue may be stopped when a different state required for the cognitive task is detected.
v. Spatial stimulus location. This may be used e.g. in some haptic embodiments with multiple locations of haptic stimulus delivery. For example, the digital hand utensil may have more than two vibrators disposed at different locations. The sequential activation of a haptic stimulus at (different) locations on the skin may emulate a "stroking feeling" which has a distinct effect on emotional valence and/or arousal.
vi. Complex sequences of acoustic/auditory information which may consist of the same parameters, but which then vary over time (e.g., a section of music). In the case of music, the properties of tempo, fundamental frequency and amplitude may still apply. Such stimuli may be able to affect the user's cognitive state beyond just arousal.

Example cognitive task types may be repeated with example states required for the cognitive task and/or desired stimulus parameters. For example, a cognitive task type for note taking may be "focused" with a repetition frequency of 15 Hz. As another example, a cognitive task type for creative writing may be "relaxed" with a repetition frequency of 1 Hz. As another example, a cognitive task type for technical subject writing may be "focused" with a repetition frequency of 15 Hz.

Exact stimuli amplitude and other properties which are preferred by the user may be identified in a testing phase to create stimuli options which have advantageous properties for the user.

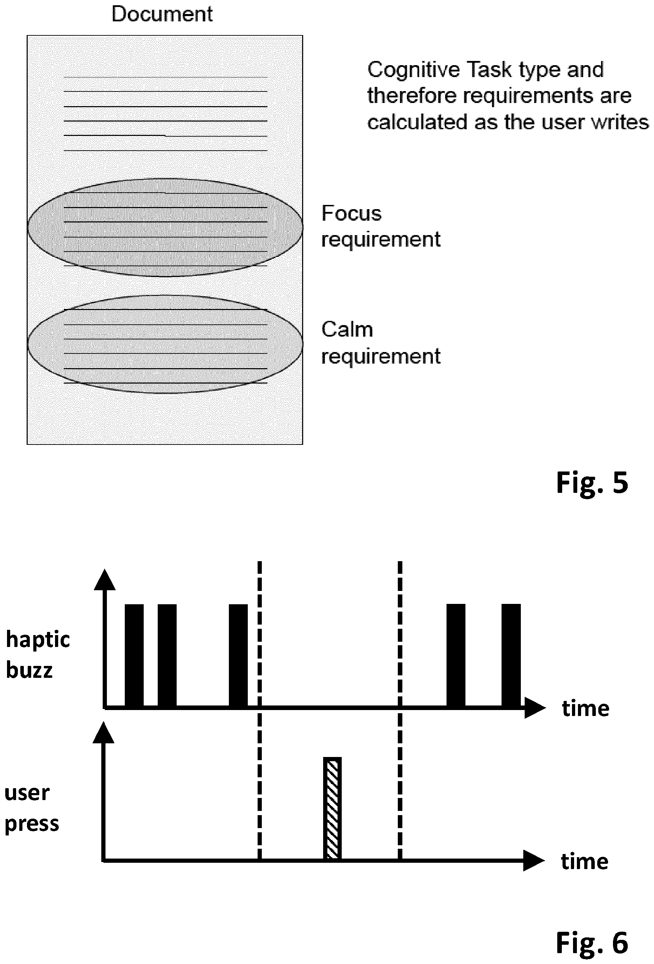

As an example, the cognitive task system 200 may be a writing system. The cognitive task may comprise a writing task and the cognitive task data may comprise writing data. An example writing task is schematically illustrated in Fig. 5. The document displayed therein also illustrates that the writing task ― and, in general, the cognitive task ― may change in the course of performing the cognitive task in that it has different portions with possibly different cognitive requirements. As an example, such differences of cognitive requirements (that is different states required for the cognitive task) may be accounted for by means of the cognitive task type. For example, when as e.g. in **Fig. 5** a focus requirement is detected (132) in a portion of the writing task/cognitive task, a cognitive state cue for enhancing the focus may be administered. Later on, when again as e.g. in **Fig. 5** a calm requirement is detected (132) in another portion of the writing task/cognitive task, a more soothing cognitive state cue may be administered.

Alternatively, or in addition the cognitive task system 200 may be a drawing system. The cognitive task may comprise a drawing task and the cognitive task data may comprise drawing data. The cognitive task system 200 may be both a writing system and a drawing system. The cognitive task may comprise both a writing task and a drawing task. The cognitive task data may comprise both writing data and drawing data.

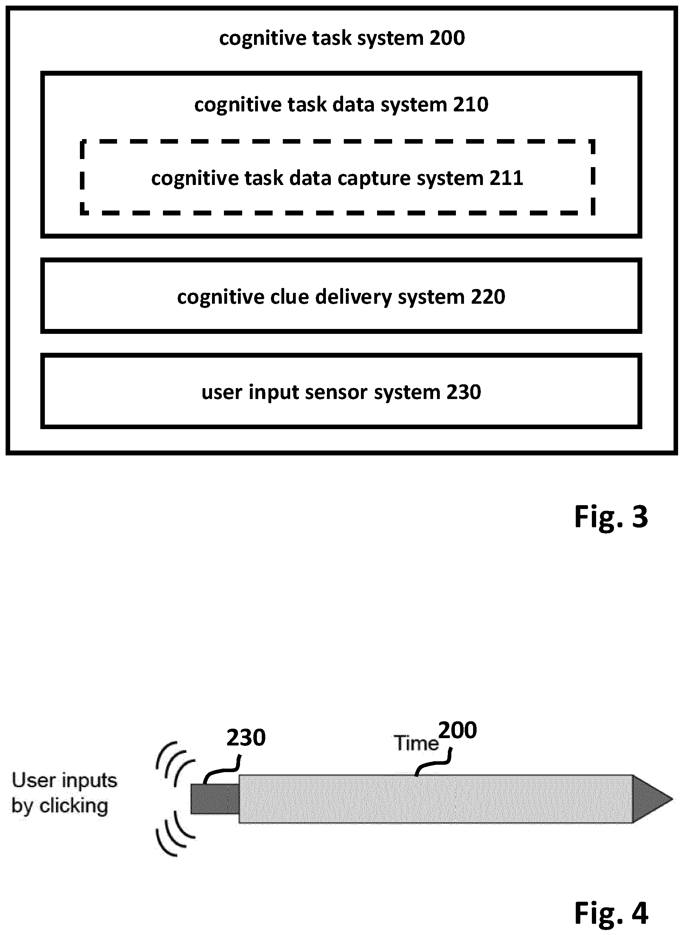

The cognitive task data may be obtained 110 by a cognitive task data system 210 of the cognitive task system 200, see e.g. **Fig. 3**.

Obtaining 110 the cognitive task data may comprise capturing 109 the cognitive task data via the cognitive task system 200. As an example, and as e.g. in **Fig. 3**, the cognitive task data may be captured 109 via a cognitive task data capture system 211 of the cognitive task system 200. Capturing 109 the cognitive task data may enable the user to write and/or draw information digitally.

Capturing 109 the cognitive task data may comprise recording one or more locations of the user's input relative to and/or within a surface of the cognitive task system 200. Capturing 109 the cognitive task data may comprise recording one or more locations of the user's input relative to and/or within a surface of the cognitive task system 200. The surface of the cognitive task system 200 may be configured to be written on and/or drawn on by the user of the cognitive task system 200.

Capturing 109 the cognitive task data may comprise recording further input data. For example, the further input data may comprise a color, a pressure, and/or a tone, e.g. associated to each recorded location of the user's input.

Capturing 109 the cognitive task data may comprise recording temporal information corresponding to the cognitive task data. For example, for each recorded location a corresponding timestamp and/or a hold time may be recorded too.

The cognitive task data may comprise a sequence of locations relative to and/or within a surface of the cognitive task system 200. Alternatively, or in addition, the cognitive task data may comprise a time series of locations relative to and/or within the surface of the cognitive task system 200.

The time series of locations may comprise temporal information as to when locations where visited. The temporal information may be used in administering/providing the (appropriate) cognitive state cue to the user of the cognitive task system 200.

As an example, the one or more locations may each be given in terms of coordinates relative to and/or within the surface of the cognitive task system 200. In case of an analog-to-digital system (A2D), the surface may be a virtual surface e.g. representing a sheet of paper. In case of a digital-to-digital system (D2D), the surface may be a surface of the digital-to-digital system (D2D) such as e.g. the surface of a touchscreen. In this case, the coordinates may be (pixel) coordinates of e.g. the touchscreen.

The method 100 may comprise recognizing 120 text based on the cognitive task data, thereby generating text data. In this case, determining 130 the cognitive state cue to be administered to the user may be (further) based on the text data. Such a scenario is schematically illustrated in **Fig. 2**.

Recognizing 120 the text based on the cognitive task data may comprise applying the cognitive task data such as e.g. the writing data to a text recognition algorithm configured to recognize text in the cognitive task data. In other words, the text recognition algorithm may output an ordered set of words representing the contents of the writing data. The text recognition algorithm may be a machine learning algorithm configured and pre-trained for recognizing the text.

The text data may comprise (or be) character encoded text such as e.g. a bit or byte sequence in terms of a character encoding (e.g. ASCII, Unicode, etc.).

**Fig. 1c** schematically illustrates embodiments of step 130 of the computer-implemented method 100, to be discussed in the following.

Determining 130 the cognitive state cue to be administered to the user based on the cognitive task data may comprise specifying 131 the cognitive state cue instruction based on the cognitive task data.

Determining 130 the cognitive state cue to be administered to the user based on the cognitive task data may comprise detecting 132 a cognitive task type based on the cognitive task data. Specifying 131 the cognitive state cue instruction may be (further) based on the cognitive task type. For example, specifying 131 the cognitive state cue instruction may be based on the cognitive task type. In other examples, specifying 131 the cognitive state cue instruction may be based on the cognitive task data and the cognitive task type.

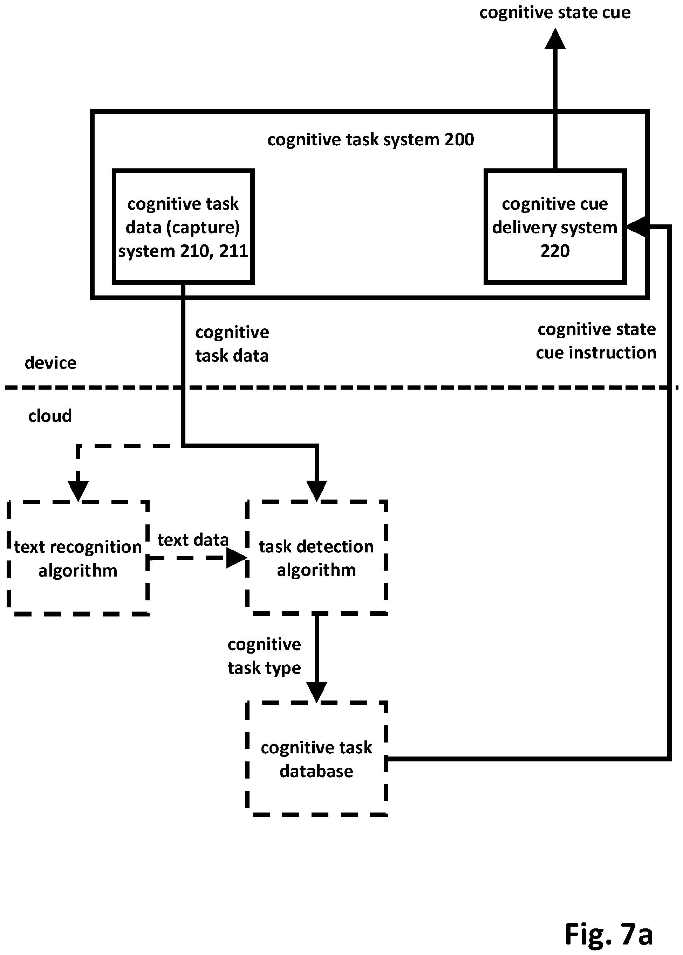

Detecting 132 the cognitive task type may comprise applying the cognitive task data to a machine learning algorithm configured and pre-trained for classifying the cognitive task data into cognitive task types, thereby detecting 132 the cognitive task type (e.g. in case of a drawing task). The machine learning algorithm may be referred to as task detection algorithm, see e.g. **Fig. 7a****-b.**

Detecting 132 the cognitive task type may comprise applying the cognitive task data and/or the text data to a machine learning algorithm configured and pre-trained for classifying the cognitive task data and/or the text data into cognitive task types, thereby detecting 132 the cognitive task type.

For example, detecting 132 the cognitive task type may comprise applying the text data to a machine learning algorithm configured and pre-trained for classifying the text data into cognitive task types, thereby detecting 132 the cognitive task type. In examples, detecting 132 the cognitive task type may comprise applying the cognitive task data and the text data to a machine learning algorithm configured and pre-trained for classifying the cognitive task data and the text data into cognitive task types, thereby detecting 132 the cognitive task type.

Determining 130 the cognitive state cue to be administered to the user based on the cognitive task data may comprise detecting 133 an emotional state of the user based on the cognitive task data and/or the cognitive task type. In this case, specifying 131 the cognitive state cue instruction may be (further) based on the emotional state of the user. Taking into account the emotional state of the user may lead to a more accurate analysis, thereby providing a more suited cognitive state cue to the user.

For example, the emotional state of the user may be detected 133 based on the cognitive task data. In other examples, the emotional state of the user may be detected 133 based on the cognitive task and the cognitive task type.

Furthermore, specifying 131 the cognitive state cue instruction may be based on the emotional state of the user. In examples, specifying 131 the cognitive state cue instruction may be based on the emotional state of the user and the cognitive state type. Specifying 131 the cognitive state cue instruction may be based on the emotional state of the user, the cognitive state type, and the cognitive state data.

Detecting 133 the emotional state of the user may comprise applying the cognitive task data and/or the cognitive task type to a machine learning algorithm configured and pre-trained for classifying the cognitive task data into emotional states, thereby detecting 133 the emotional state of the user.

The machine learning algorithm may e.g. be referred to as emotional state detection algorithm. For example, detecting 133 the emotional state of the user may comprise applying the cognitive task data to a machine learning algorithm configured and pre-trained for classifying the cognitive task data into emotional states. As another example, detecting 133 the emotional state of the user may comprise applying the cognitive task data and the cognitive task type to a machine learning algorithm configured and pre-trained for classifying the cognitive task data (and the cognitive task type) into emotional states.

Furthermore, the method 100 may comprise detecting an emotional valence of the user's (current) cognitive task e.g. by means of a tone and/or sentiment detection algorithm. This may be useful for creative (writing) tasks.

Specifying 131 the cognitive state cue instruction may comprise selecting 134 a predetermined cognitive state cue instruction based on the cognitive task data, the cognitive task type, and/or the emotional state of the user, thereby generating the cognitive state cue instruction.

For example, selecting 134 the predetermined cognitive state cue instruction may be based on the cognitive task data. Alternatively, or in addition, selecting 134 the predetermined cognitive state cue instruction may be based on the cognitive task type. Alternatively, or in addition, selecting 134 the predetermined cognitive state cue instruction may be based on the emotional state of the user. As an example, selecting 134 the predetermined cognitive state cue instruction may be based on the cognitive task type and the emotional state of the user.

Selecting 134 the predetermined cognitive state cue instruction may comprise retrieving 135 a predetermined cognitive state cue instruction corresponding to the cognitive task data, the cognitive task type, and/or the emotional state of the user from a database, thereby selecting 134 the predetermined cognitive state cue instruction.

For example, the predetermined cognitive state cue instruction retrieved 135 from the database may correspond to the cognitive task data. Alternatively, or in addition, the predetermined cognitive state cue instruction retrieved 135 from the database may correspond to the cognitive task type. Such a scenario is illustrated in **Fig. 7a****.** Alternatively, or in addition, the predetermined cognitive state cue instruction retrieved 135 from the database may correspond to the emotional state of the user. As an example, the predetermined cognitive state cue instruction retrieved 135 from the database may correspond to the cognitive task type and the emotional state of the user. For example, the database may be the cognitive task database of **Fig. 7a****-b.**

For example, the database may be configured to store the possible cognitive task types which are associated with different desired cognitive states (e.g. referred to as states required for the cognitive task) and known stimulus properties which achieves such a state (e.g. referred to as the desired stimulus parameters).

Specifying 131 the cognitive state cue instruction may comprise parametrizing 136 the predetermined cognitive state cue instruction based on the cognitive task data, the cognitive task type, and/or the emotional state of the user, thereby updating the cognitive state cue instruction.

For example, the predetermined cognitive state cue instruction may be parametrized 136 based on the cognitive task data. Alternatively, or in addition, the predetermined cognitive state cue instruction may be parametrized 136 based on the cognitive task type. Alternatively, or in addition, the predetermined cognitive state cue instruction may be parametrized 136 based on the emotional state of the user. As an example, the predetermined cognitive state cue instruction may be parametrized 136 based on the cognitive task type and the emotional state of the user.

Parametrizing 136 the predetermined cognitive state cue instruction may comprise retrieving 137 one or more parameters corresponding to the cognitive task data, the cognitive task type, and/or the emotional state of the user from a database. The one or more parameters may be used in parametrizing 136 the predetermined cognitive state cue instruction. The one or more parameters may be the desired stimulus parameters.

For example, the one or more parameters retrieved 137 from the database may correspond to the cognitive task data. Alternatively, or in addition, the one or more parameters retrieved 137 from the database may correspond to the cognitive task type. Alternatively, or in addition, the one or more parameters retrieved 137 from the database may correspond to the emotional state of the user. As an example, the one or more parameters retrieved 137 from the database may correspond to the cognitive task type and the emotional state of the user. For example, the database may be the cognitive task database of **Fig. 7a****-b.**

The cognitive state cue instruction may be executed 140 by a cognitive cue delivery system 220 of the cognitive task system 200, as e.g. displayed in **Fig. 3****.**

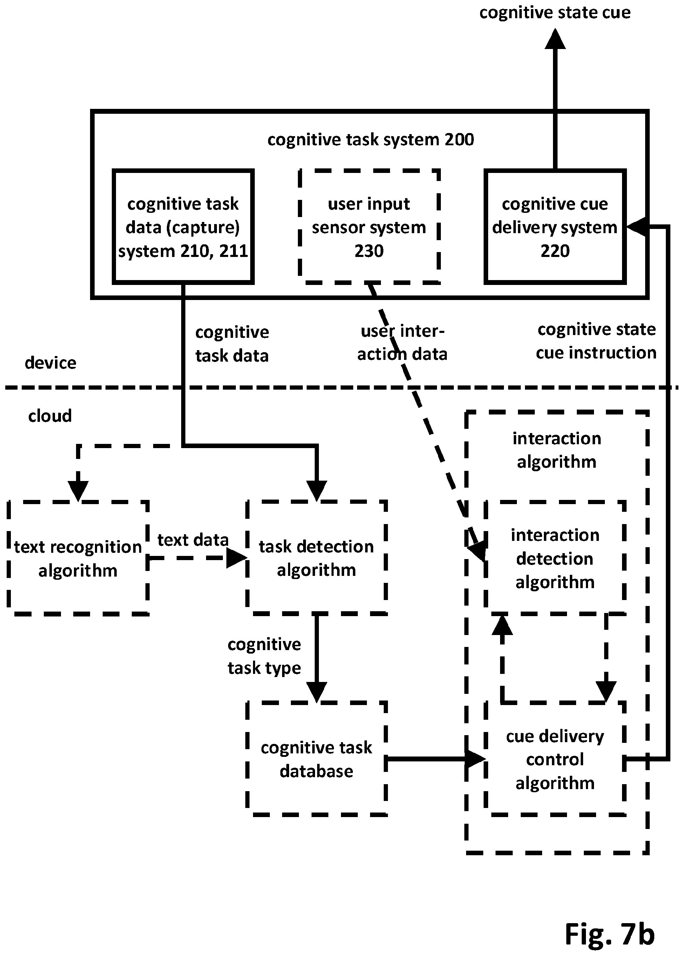

The method 100 may comprise obtaining 150, e.g. via a user input sensor system 230 of the cognitive task system 200, user interaction data. This is e.g. displayed in **Fig. 3** and **Fig. 7b****.**

Determining 130 the cognitive state cue to be administered to the user may be (further) based on the user interaction data. For example, the user of the cognitive task system 200 may interact in order to indicate contentment or annoyance regarding the previous cognitive state cue.

Executing 140 the cognitive state cue instruction may be based on the user interaction data. For example, a point in time for executing 140 the cognitive state cue instruction may depend on the user interaction data.

The method 100 may comprise recognizing 151 a user interaction based on the user interaction data, thereby generating a user interaction event. The method 100 may further comprise executing 152 a further cognitive state cue instruction after a predetermined time interval, if the user interaction event occurs within a predetermined time interval. Such a scenario is illustrated in **Fig. 6**, wherein an example user interaction event consists of a single user press within the predetermined time interval

Recognizing 151 the user interaction may comprise recognizing a requested user interaction, thereby generating a requested user interaction event (again being a user interaction event). For example, the user may be requested to interact with the user input sensor system 230 in a predetermined pattern. In this case, the user interaction may comprise e.g. a predetermined rhythm that may be recognized 151, thereby generating the requested user interaction event. The user may be taught as to what user interaction is requested. As an example, the predetermined rhythm that the user is requested to apply to the user input sensor system 230 may be administered by the cognitive cue delivery system 220.

The user interaction may be a physical user interaction. For example, and as displayed in **Fig. 4**, such a physical user interaction may comprise clicking on the digital hand utensil. The user interaction event may comprise a user interaction pattern (e.g. in a predetermined rhythm).

The further cognitive state cue instruction may be the cognitive state cue instruction. For example, in this case the cognitive state cue instruction may be repeated after the predetermined time interval.

In examples, the further cognitive state cue instruction may be the cognitive state cue instruction under the proviso that the computer-implemented method 100 for administering a cognitive state cue to the user of the cognitive task system 200 is run at a further point in time.

The user interaction may be used to retrain the one or more machine learning algorithms of the method 100, the cognitive task system 200, and/or the computer system, thereby adjusting the method and systems to individual needs of the user.

As an example, active user haptic participation or, more generally, active user interaction (e.g. also referred to as fidgeting) may be enabled by the computer system and the cognitive task system 200 displayed in **Fig. 7b**. **Fig. 6** shows an example embodiment of cognitive state cue(s) with desired stimulus parameters. To enable active user engagement in the cognitive state cue, the user input sensor system may be included as part of the cognitive task system to detect user interaction. This may e,g, comprise one or more accelerometers to detect tapping of the pen against a surface. These accelerometers may be the same as those of the cognitive task data capture system 211. In case of active user interaction being enabled by clicking/pressing the digital hand utensil as e.g. in **Fig. 4**, the user input sensor system may, for example, comprise a contact sensor to detect clicks of the digital hand utensil.

An algorithm (e.g. referred to as interaction (detection) algorithm) may be required to process the user interaction data to detect a user interaction event. Furthermore, an algorithm (e.g. referred to as the cue delivery control algorithm) may control the delivery of the desired stimulus properties which may be based on a detected user interaction. In **Fig. 7b** the cue delivery control algorithm may e.g. send an expected user input to the interaction detection algorithm. The interaction detection algorithm may send a user input flag to the cue delivery control algorithm.

The desired stimulus parameters may additionally include a parameter of user response requirements which act as 'gates' for the continuation of the cognitive state cue. For example, a response requirement may consist of two time values between which a specific user input must be detected for the cognitive state cue to repeat or continue. This is demonstrated in **Fig. 6**. For example, an entire (predetermined) pattern may be delivered at least once without the requirement for user input in order to establish the expected pattern.

The method 100 may comprise predicting 160 a future portion of the user's cognitive task, thereby generating predicted future data.

Determining 130 the cognitive state cue to be administered to the user may be (further) based on the predicted future data.

Executing 140 the cognitive state cue instruction may be (further) based on the predicted future data.

In case of generating the text data, predicting 160 the future portion of the user's cognitive task may comprise applying the text data to a machine learning algorithm configured and pre-trained for continuing the text data, thereby generating the predicted future data.

The machine learning algorithm may e.g. be referred to as text prediction algorithm. In this case, the predicted future data may be of the same type as the text data. The machine learning algorithm may comprise (or be) an autoregressive language model configured to generate human-like text. An example of such an autoregressive language model may be Generative Pre-trained Transformer 3 (GPT-3).

As an example, the proposed computer system and/or cognitive task system 200 may be used as part of a writing aide, wherein the text prediction algorithm is included to predict text which the user may write next, using the text data generated so far. Here, the task detection algorithm may then be used on the text data and/or the predicted section of the text. In so doing, user cognitive states may be promoted which may be useful in the subsequent writing task. This may reduce or remove a time lag that would exist without predicting the text continuation, that is when merely analyzing existing written text data to create a response. The writing aid system's suggestion for the emotional and/or arousal properties of the subsequent section of text may be communicated to the user.

The method 100 may comprise running 170 the computer-implemented method 100 for administering a cognitive state cue to the user of the cognitive task system 200 at a further point in time. In so doing, cognitive state cues may be administered to the user of the cognitive task system 200 continuously and/or over a period of time.

Any of the pre-trained machine learning algorithms disclosed herein may have been trained on appropriate training datasets and corresponding labels (supervised learning). The method 100 may comprise re-training any of the pre-trained machine learning algorithms e.g. based on user interaction data.

There is disclosed a cognitive task system 200 for administering a cognitive state cue to a user of the cognitive task system 200. The cognitive task system 200 comprises a cognitive task data system 210 configured to obtain 110 cognitive task data while the user performs a cognitive task. The cognitive task system 200 further comprises a cognitive cue delivery system 220 configured to execute 140 a cognitive state cue instruction, thereby administering the cognitive state cue to the user. The cognitive state cue instruction may be generated by determining 130 the cognitive state cue to be administered to the user based on the cognitive task data. The cognitive task system 200 is schematically illustrated in **Fig. 3**.

The cognitive task system 200 may be configured to run the computer-implemented method 100 for administering a cognitive state cue to the user of the cognitive task system 200.

The cognitive task data system 210 may comprise a cognitive task data capture system 211 configured to capture 109 the cognitive task data while the user performs the cognitive task. In other words, the cognitive task data capture system 211 may be configured to record the location of user input relative to a surface enabling the user to input/write information digitally. The cognitive task data capture system 211 may comprise an analog-to-digital system. As an example, the analog-to-digital system may comprise a digital hand utensil. **Fig. 4** may show an example digital hand utensil.

For example, the digital hand utensil may be a digital pen. Alternatively, or in addition, the digital hand utensil may be a smart pen. Alternatively, or in addition, the digital hand utensil may be a smart pencil. Alternatively, or in addition, the digital hand utensil may be a smart brush. The digital hand utensil may be equipped with one or more sensors (e.g. one or more accelerometers, touch sensor, pressure sensor etc.) to record the digital hand utensil's movement from one location of the surface of the cognitive task system 200 to another location of the surface of the cognitive task system 200. The digital hand utensil may or may not leave a trace (e.g. on a sheet of paper) on the surface of the surface of the cognitive task system 200.

The cognitive task data capture system 211 may comprise a digital-to-digital system. As an example, the digital-to-digital system may comprise a touch screen.

For example, the digital-to-digital (D2D) system may be a tablet. Alternatively, or in addition, the digital-to-digital (D2D) system may be a smartphone. Alternatively, or in addition, the digital-to-digital (D2D) system may be a slate. Alternatively, or in addition, the digital-to-digital (D2D) system may be an e-writer. In general, the digital-to-digital system (D2D) may comprise (or be) a screen configured to record one or more locations input from a finger and/or stylus.

The cognitive cue delivery system 220 may comprise a vibrator configured to provide haptic feedback to the user. As an example, the digital hand utensil may comprise the vibrator. As another example, the touchscreen may be configured to deliver haptic feedback to the finger or the stylus. Alternatively, or in addition, the cognitive cue delivery system 220 may comprise a speaker configured to provide acoustic feedback to the user. Other feedback means may be feasible too.

The cognitive task system 200 may comprise a user input sensor system 230 configured to obtain 150 user interaction data. The user input sensor system 230 may comprise at least one sensor.

For example, the at least one sensor may comprise (or be) one or more accelerometers (e.g. of the digital hand utensil). The one or more accelerometers may be the ones of the cognitive task data capture system 211. Alternatively, or in addition, the at least one sensor may comprise (or be) a contact sensor (e.g. of the touch screen). Alternatively, or in addition, the at least one sensor may comprise (or be) a pressure sensor. Other sensors may be feasible too. The cognitive task data capture system 211 and the user input sensor system 230 may share the same sensors (as e.g. in the case of a touchscreen).

There is disclosed a computer system configured to execute the computer-implemented method 100 for administering a cognitive state cue to the user of the cognitive task system 200. The computer system may comprise at least one processor such as e.g. a (C)PU and a memory such as e.g. RAM. The computer system may further comprise a storage such as e.g. HDD or SDD. The computer system may be configured for data exchange with a (cloud) server.

The cognitive task system 200 may comprise or be the computer system. On the other hand, the computer system may comprise the cognitive task system 200. The computer system may comprise a cloud server, as e.g. in **Fig. 7a****-b**. In this case, the cognitive task system 200 and the cloud server may be configured in a network to communicate with each other and/or for data exchange according to a given protocol. Any algorithmic step of the computer-implemented method 100 may be implemented on the cloud server rather than in the cognitive task system 200. For example, any machine learning algorithm may be implemented on the cloud server. In fact, the cloud server may have better computing resources and/or more storage than the cognitive task system 200. For example, the text recognition algorithm may be implemented in the cloud server, as e.g. in **Fig. 7a****-b**. Alternatively, or in addition, the task detection algorithm may be implemented on the cloud server. Alternatively, or in addition, the emotional state detection algorithm may be implemented on the cloud server. Alternatively, or in addition, the text prediction algorithm may be implemented on the cloud server. Also, as e.g. in **Fig. 7a****-b,** the cognitive task database may be implemented on the cloud server.

There is disclosed a computer program configured to execute the computer-implemented method 100 for administering a cognitive state cue to the user of the cognitive task system 200. The computer program may be in interpretable or compiled form. The computer program or portions thereof may be loaded as a bit or byte sequence into the RAM of the computer system and/or of the cognitive task system 200.

There is disclosed a computer-readable medium or signal storing the computer program. The medium may be e.g. one of RAM, ROM, EPROM, HDD, SDD etc. storing the computer program.

Although the present invention has been described above and is defined in the attached claims, it should be understood that the invention may alternatively be defined in accordance with the following embodiments:
1. A computer-implemented method (100) for administering a cognitive state cue to a user of a cognitive task system (200), comprising:
   - obtaining (110) cognitive task data while the user performs a cognitive task;
   - determining (130) the cognitive state cue to be administered to the user based on the cognitive task data, thereby generating a cognitive state cue instruction;
   - executing (140) the cognitive state cue instruction, thereby administering, via the cognitive task system (200), the cognitive state cue to the user.
2. The method (100) of embodiment 1, wherein the cognitive state cue comprises a stimulus promoting a cognitive state of the user of the cognitive task system.
3. The method (100) of embodiment 1 or 2, wherein administering the cognitive state cue to the user comprises providing haptic feedback to the user.
4. The method (100) of one of the preceding embodiments, wherein administering the cognitive state cue to the user comprises providing acoustic feedback to the user.
5. The method (100) of one of the preceding embodiments, wherein the cognitive task system (200) is a writing system.
6. The method (100) of one of the preceding embodiments, wherein the cognitive task comprises a writing task and the cognitive task data comprises writing data.
7. The method (100) of one of the preceding embodiments, wherein the cognitive task system (200) is a drawing system.
8. The method (100) of one of the preceding embodiments, wherein the cognitive task comprises a drawing task and the cognitive task data comprises drawing data.
9. The method (100) of one of the preceding embodiments, wherein obtaining (110) the cognitive task data comprises capturing (109) the cognitive task data via the cognitive task system (200).
10. The method (100) of embodiment 9, wherein capturing (109) the cognitive task data comprises recording one or more locations of the user's input relative to and/or within a surface of the cognitive task system (200).
11. The method (100) of one of the preceding embodiments, wherein the cognitive task data comprises a sequence of locations relative to and/or within a surface of the cognitive task system (200).
12. The method (100) of one of the preceding embodiments, wherein the cognitive task data comprises a time series of locations relative to and/or within the surface of the cognitive task system 200.
13. The method (100) of one of the preceding embodiments, comprising:
   - recognizing (120) text based on the cognitive task data, thereby generating text data; wherein determining (130) the cognitive state cue to be administered to the user is based on the text data.
14. The method (100) of one of the preceding embodiments, wherein determining (130) the cognitive state cue to be administered to the user based on the cognitive task data comprises:
   - specifying (131) the cognitive state cue instruction based on the cognitive task data.
15. The method (100) of embodiment 14, wherein determining (130) the cognitive state cue to be administered to the user based on the cognitive task data comprises:
   - detecting (132) a cognitive task type based on the cognitive task data;
   wherein specifying (131) the cognitive state cue instruction is based on the cognitive task type.
16. The method (100) of embodiment 15, wherein detecting (132) the cognitive task type comprises applying the cognitive task data to a machine learning algorithm configured and pre-trained for classifying the cognitive task data into cognitive task types, thereby detecting (132) the cognitive task type.
17. The method (100) of embodiment 15 or 16, when dependent on embodiment 13, wherein detecting (132) the cognitive task type comprises applying the cognitive task data and/or the text data to a machine learning algorithm configured and pre-trained for classifying the cognitive task data and/or the text data into cognitive task types, thereby detecting (132) the cognitive task type.
18. The method (100) of one of the embodiments 14 to 17, wherein determining (130) the cognitive state cue to be administered to the user based on the cognitive task data comprises:
   - detecting (133) an emotional state of the user based on the cognitive task data and/or the cognitive task type;
   wherein specifying (131) the cognitive state cue instruction is based on the emotional state of the user.
19. The method (100) of embodiment 18, wherein detecting (133) the emotional state of the user comprises applying the cognitive task data and/or the cognitive task type to a machine learning algorithm configured and pre-trained for classifying the cognitive task data into emotional states, thereby detecting (133) the emotional state of the user.
20. The method (100) of one of the embodiments 14 to 19, wherein specifying (131) the cognitive state cue instruction comprises:
   - selecting (134) a predetermined cognitive state cue instruction based on the cognitive task data, the cognitive task type, and/or the emotional state of the user;
   thereby generating the cognitive state cue instruction.
21. The method (100) of embodiment 20, wherein selecting (134) the predetermined cognitive state cue instruction comprises retrieving (135) a predetermined cognitive state cue instruction corresponding to the cognitive task data, the cognitive task type, and/or the emotional state of the user from a database, thereby selecting (134) the predetermined cognitive state cue instruction.
22. The method (100) of embodiment 20 or 21, wherein specifying (131) the cognitive state cue instruction comprises:
   - parametrizing (136) the predetermined cognitive state cue instruction based on the cognitive task data, the cognitive task type, and/or the emotional state of the user;
   thereby updating the cognitive state cue instruction.
23. The method (100) of embodiment 22, wherein parametrizing (136) the predetermined cognitive state cue instruction comprises retrieving (137) one or more parameters corresponding to the cognitive task data, the cognitive task type, and/or the emotional state of the user from a database;
   wherein the one or more parameters are used in parametrizing (136) the predetermined cognitive state cue instruction.
24. The method (100) of one of the preceding embodiments, wherein the cognitive state cue instruction is executed (140) by a cognitive cue delivery system (220) of the cognitive task system (200).
25. The method (100) of one of the preceding embodiments, comprising:
   - obtaining (150), via a user input sensor system (230) of the cognitive task system (200), user interaction data.
26. The method (100) of embodiment 25, wherein determining (130) the cognitive state cue to be administered to the user is based on the user interaction data.
27. The method (100) of embodiment 25 or 26, wherein executing (140) the cognitive state cue instruction is based on the user interaction data.
28. The method (100) of one of the embodiments 25 to 26, comprising:
   - recognizing (151) a user interaction based on the user interaction data, thereby generating a user interaction event;
   - executing (152) a further cognitive state cue instruction after a predetermined time interval, if the user interaction event occurs within a predetermined time interval.
29. The method (100) of embodiment 28, wherein the further cognitive state cue instruction is the cognitive state cue instruction.
30. The method (100) of embodiment 28, wherein the further cognitive state cue instruction is the cognitive state cue instruction of embodiment 1 under the proviso that the computer-implemented method (100) for administering a cognitive state cue to the user of the cognitive task system (200) is run at a further point in time.
31. The method (100) of one of the preceding embodiments, comprising:
   - predicting (160) a future portion of the user's cognitive task, thereby generating predicted future data.
32. The method (100) of embodiment 31, wherein determining (130) the cognitive state cue to be administered to the user is based on the predicted future data.
33. The method (100) of embodiment 31 or 32, wherein executing (140) the cognitive state cue instruction is based on the predicted future data.
34. The method (100) of one of the embodiments 31 to 33, when dependent on embodiment 13, wherein predicting (160) the future portion of the user's cognitive task comprises applying the text data to a machine learning algorithm configured and pre-trained for continuing the text data, thereby generating the predicted future data.
35. The method (100) of one of the preceding embodiments, comprising:
   - running (170) the computer-implemented method (100) for administering a cognitive state cue to the user of the cognitive task system (200) at a further point in time.
36. A cognitive task system (200) for administering a cognitive state cue to a user of the cognitive task system (200), comprising:
   - a cognitive task data system (210) configured to obtain (110) cognitive task data while the user performs a cognitive task;
   - a cognitive cue delivery system (220) configured to execute (140) a cognitive state cue instruction, thereby administering the cognitive state cue to the user;
   wherein the cognitive state cue instruction is generated by determining (130) the cognitive state cue to be administered to the user based on the cognitive task data.
37. The cognitive task system (200) of embodiment 36, configured to run the computer-implemented method (100) for administering a cognitive state cue to the user of the cognitive task system (200).
38. The cognitive task system (200) of embodiment 36 or 37, wherein the cognitive task data system (210) comprises a cognitive task data capture system (211) configured to capture (109) the cognitive task data while the user performs the cognitive task.
39. The cognitive task system (200) of embodiment 38, wherein the cognitive task data capture system (211) comprises an analog-to-digital system.
40. The cognitive task system (200) of embodiment 39, wherein the analog-to-digital system comprises a digital hand utensil.
41. The cognitive task system (200) of one of the embodiments 38 to 40, wherein the cognitive task data capture system (211) comprises a digital-to-digital system.
42. The cognitive task system (200) of embodiment 41, wherein the digital-to-digital system comprises a touch screen.
43. The cognitive task system (200) of one of the embodiments 36 to 42, wherein the cognitive cue delivery system 220 comprises a vibrator configured to provide haptic feedback to the user.
44. The cognitive task system (200) of one of the embodiments 36 to 43, wherein the cognitive cue delivery system 220 comprises a speaker configured to provide acoustic feedback to the user.
45. The cognitive task system (200) of one of the embodiments 36 to 44, comprising:
   - a user input sensor system (230) configured to obtain (150) user interaction data.
46. The cognitive task system (200) of embodiment 45, wherein the user input sensor system (230) comprises at least one sensor.
47. A computer system configured to execute the computer-implemented method (100) for administering a cognitive state cue to the user of the cognitive task system (200) according to one of the embodiments 1 to 35.
48. A computer program configured to execute the computer-implemented method (100) for administering a cognitive state cue to the user of the cognitive task system (200) according to one of the embodiments 1 to 35.
49. A computer-readable medium or signal storing the computer program of embodiment 48.

## Claims

1. A computer-implemented method (100) for administering a cognitive state cue to a user of a cognitive task system (200), comprising:
- obtaining (110) cognitive task data while the user performs a cognitive task;
- determining (130) the cognitive state cue to be administered to the user based on the cognitive task data, thereby generating a cognitive state cue instruction;
- executing (140) the cognitive state cue instruction, thereby administering, via the cognitive task system (200), the cognitive state cue to the user.

2. The method (100) of claim 1, wherein the cognitive state cue comprises a stimulus promoting a cognitive state of the user of the cognitive task system.

3. The method (100) of claim 1 or 2, wherein administering the cognitive state cue to the user comprises providing haptic feedback and/or acoustic feedback to the user.

4. The method (100) of one of the preceding claims, wherein obtaining (110) the cognitive task data comprises capturing (109) the cognitive task data via the cognitive task system (200).

5. The method (100) of one of the preceding claims, comprising:
- recognizing (120) text based on the cognitive task data, thereby generating text data; wherein determining (130) the cognitive state cue to be administered to the user is based on the text data.

6. The method (100) of one of the preceding claims, wherein determining (130) the cognitive state cue to be administered to the user based on the cognitive task data comprises:
- specifying (131) the cognitive state cue instruction based on the cognitive task data;
- detecting (132) a cognitive task type based on the cognitive task data;
wherein specifying (131) the cognitive state cue instruction is based on the cognitive task type.

7. The method (100) of claim 6, wherein determining (130) the cognitive state cue to be administered to the user based on the cognitive task data comprises:
- detecting (133) an emotional state of the user based on the cognitive task data and/or the cognitive task type;
wherein specifying (131) the cognitive state cue instruction is based on the emotional state of the user.

8. The method (100) of one of the preceding claims, comprising:
- obtaining (150), via a user input sensor system (230) of the cognitive task system (200), user interaction data.

9. The method (100) of claim 8, wherein determining (130) the cognitive state cue to be administered to the user and/or executing (140) the cognitive state cue instruction is based on the user interaction data.

10. The method (100) of claim 8 or 9, comprising:
- recognizing (151) a user interaction based on the user interaction data, thereby generating a user interaction event;
- executing (152) a further cognitive state cue instruction after a predetermined time interval, if the user interaction event occurs within a predetermined time interval.

11. The method (100) of one of the preceding claims, comprising:
- predicting (160) a future portion of the user's cognitive task, thereby generating predicted future data.

12. The method (100) of claim 11, wherein determining (130) the cognitive state cue to be administered to the user and/or executing (140) the cognitive state cue instruction is based on the predicted future data.

13. The method (100) of claim 11 or 12, when dependent on claim 5, wherein predicting (160) the future portion of the user's cognitive task comprises applying the text data to a machine learning algorithm configured and pre-trained for continuing the text data, thereby generating the predicted future data.

14. A cognitive task system (200) for administering a cognitive state cue to a user of the cognitive task system (200), comprising:
- a cognitive task data system (210) configured to obtain (110) cognitive task data while the user performs a cognitive task;
- a cognitive cue delivery system (220) configured to execute (140) a cognitive state cue instruction, thereby administering the cognitive state cue to the user;
wherein the cognitive state cue instruction is generated by determining (130) the cognitive state cue to be administered to the user based on the cognitive task data.

15. The cognitive task system (200) of claim 14, wherein the cognitive task data system (210) comprises a cognitive task data capture system (211) configured to capture (109) the cognitive task data while the user performs the cognitive task, wherein the cognitive task data capture system (211) comprises an analog-to-digital system, wherein the analog-to-digital system comprises a digital hand utensil.
